# EUROPEAN PATENT SPECIFICATION

(11) **EP 3 027 591 B1**
(45) Date of publication and mention of the grant of the patent: **18.07.2018**
(21) Application number: 14766002.1
(22) Date of filing: 29.07.2014
(51) Int. Cl.: C07D 217/26, A61K 31/47, A61P 35/00

(54) **NOVEL DERIVATIVES OF A 3,4-DIHYDROISOQUINOLINE-3-CARBOXYLIC ACID HAVING ANTI-CANCER PROPERTIES, A METHOD FOR THEIR SYNTHESIS, PHARMACEUTICAL COMPOSITIONS COMPRISING SAID DERIVATIVES, AND THEIR USE**
NEUE DERIVATE VON 3,4-DIHYDROISOCHINOLIN-3-CARBONSÄURE MIT KREBSHEMMENDER WIRKUNG, EIN VERFAHREN ZUR DEREN HERSTELLUNG, DIE PHARMAZEUTISCHE ZUSAMMENSETZUNGEN ENTHALTEND DIESE DERIVATE UND IHRE VERWENDUNG
NOUVEAUX DÉRIVÉS D'UN ACIDE 3,4-DIHYDROISOQUINOLINE-3-CARBOXYLIQUE AYANT DES PROPRIÉTÉS ANTICANCÉREUSES, LEUR PROCÉDÉ DE SYNTHÈSE, COMPOSITIONS PHARMACEUTIQUES COMPRENANT LESDITS DÉRIVÉS, ET LEUR UTILISATION

(30) Priority: 30.07.2013 PL 40492013
(43) Date of publication of application: 08.06.2016
(73) Proprietor: Narodowy Instytut Zdrowia Publicznego Panstwowy Zaklad Higieny, 00-791 Warszawa (PL); Uniwersytet Przyrodniczo-Humanistyczny W Siedlcach, 08-110 Siedlce (PL)
(72) Inventor: SOLECKA, Jolanta, PL-04-758 Warszawa (PL); KAWECKI, Robert, PL-03-472 Warszawa (PL); POSTEK, Magdalena, PL-05-092 Lomianki (PL); PYPOWSKI, Krzysztof, PL-08-110 Siedlce (PL); ZIEMSKA, Joanna, PL-01-745 Warszawa (PL); GUSPIEL, Adam, PL-34-424 Banska Nizna (PL)
(74) Representative: Dargiewicz, Joanna
(86) International application number: PCT/IB2014/063518
(87) International publication number: WO 2015/015420

(56) References cited:
- WO-A1-92/03419
- US-A1- 2010 267 767
- JAEBONG JANG ET AL: "Asymmetric formal synthesis of schulzeines A and C", ORGANIC & BIOMOLECULAR CHEMISTRY, vol. 10, no. 27, 1 January 2012 (2012-01-01), page 5202, XP055147868, ISSN: 1477-0520, DOI: 10.1039/c2ob25772f
- F.N.ALVAREZ ET AL: "6,7-Dihydroxy-3,4-Dihydroisoquinoline: A Novel Inhibitor of Nuclear Factor-kappaB and in vitro Invasion in Murine Mammary Cancer Cells", CHEMOTHERAPY, vol. 55, 30 April 2009 (2009-04-30), pages 175-182, XP009180893, cited in the application
- BERMEJO A ET AL: "Syntheses and Antitumor Targeting G1 Phase of the Cell Cycle of Benzoyldihydroisoquinolines and Related 1-Substituted Isoquinolines", JOURNAL OF MEDICINAL CHEMISTRY, AMERICAN CHEMICAL SOCIETY, US, vol. 45, no. 23, 1 January 2002 (2002-01-01), pages 5058-5068, XP002302936, ISSN: 0022-2623, DOI: 10.1021/JM020831A

## Description

The present invention relates to new 3,4-dihydroisoquinoline derivatives, the method of preparation thereof, pharmaceutical compositions comprising 3,4-dihydroisoquinoline derivatives and to the uses of said derivatives and said compositions.

The National Cancer Registry (Krajowy Rejestr Nowotworów) in Poland in 2010 received information about 70 024 initial notifications of malignant cancer in men and 70 540 in women (over 140 thousand cancer notifications in total). The estimation of the number of cases in 2010, taking the completeness of the register into account, indicates that the number of new cases was approximately 155 thousand. For every 100 thousand of the Polish population there are 365 cases of malignant cancers. In 2010 the number of deaths caused by malignant cancer among men was around 52 thousand, and among women - around 41 thousand. For every 100 thousand of the Polish population there are 132 thereby induced deaths. In men the predominating cancers are: lung (21 percent), prostate (13 percent), colorectal (11 percent), bladder (7 percent) and stomach cancer (5 percent). The remaining 41 percent are other cancers. In case of women the major problem is: breast (23 percent), colorectal (10 percent), lung (9 percent), endometrial (7 percent), ovarian (5 percent) and cervical cancer (4 percent). The remaining cancers make up 42 percent of the cases. The authors predict that in the coming years the incidence structure will not change: the most prevalent cancers in men will remain: lung, prostate and colorectal cancer, while in women breast, colorectal, lung and endometrial cancer [Wojciechowska U., Didkowska J., Zatoński W.: Nowotwory z 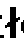 ośliwe w Polsce w 2010 r.].

The limitations in cancer chemotherapy arise mainly from the fact that the cytostatic agents used carry very severe side effects and many cancer cells exhibit acquired or congenital resistance to the administered drugs. The cause of insensitivity to chemotherapy is believed to be the numerous changes occurring on the cellular and genetic level. Therefore it is necessary to search for new, less toxic therapeutic agents and more effective therapies [Śliwińska-Hill U., Trocha J.: Najnowsze Terapie Przeciwnowotworowe, Post. Farm. 2011, 14-19].

3,4-dihydroisoquinoline derivatives exhibit a variety of interesting biological properties. Their described activities are *inter alia* anti-metastatic (Alvarez, F.N.; Carlson, L.M.; Lindner, I.; Lee, K.P.: 6,7-Dihydroxy-3,4-Dihydroisoquinoline: A Novel Inhibitor of Nuclear Factor-κB and in vitro Invasion in Murine Mammary Cancer Cells Chemotherapy 2009; 55, 175-182), analgesic and anti-inflammatory (Khalturina, V. V.; Shklyaev, Yu. V.; Makhmudov, R. R.; Maslivets, A. N.: Pharm. Chem. J. 2010, 44, 480-482), antiarrhythmic and antiaggregatory (Aleksandrov, B. B.; Gavrilov, M. S.; Dautova, R. Z.: Biological activity of fluorinated 3,4-dihydroisoquinolines Pharm. Chem. J. 1992, 26, 57-58), hypotensive (Diana, G. D.; Hinshaw, W. B.; Lape, H. E.: Synthesis and antihypertensive activity of 1-amino-3,4-dihydroisoquinolines J. Med. Chem., 1977, 20, 449-452), antibacterial (Harmon, R. E.; Jensen, B. L., Gupta, S. K., Hanka, L. J.: Synthesis and antibacterial activity of 1-styryl-3,4-dihydroisoquinoline J. Pharm. Sci. 1970, 59, 576), antioxidative (Okumura S, Takeshita S, Enei H, Ninagawa S.: Isochinolinderivate und ihre Verwendung als Antioxidationsmittel, German patent document no. DE 2342474 and US patent document no. US 3846573, 1974). They are also catechol methyl transferase inhibitors (Cheng, B.Y.; Origitano, T.C.; Collins, M.A. J. Neurochem.: Inhibition of catechol--methyltransferase by 6,7-dihydroxy-3,4-dihydroisoquinolines related to dopamine: demonstration using liquid chromatography and a novel substrate for O-methylation 1987, 48, 779-86) and JNK3 kinase inhibitors (Christopher, J.A.; Atkinson, F.L.; Bax, B.D.; Brown, M.J.; Champigny, A.C.; Chuang, T.T.; Jones, E.J.; Mosley, J.E.; Musgrave, J.R.: 1-Aryl-3,4-dihydroisoquinoline inhibitors of JNK3 Bioorg. Med. Chem. Lett. 2009, 19, 2230-2234).

The aim of the present invention was to provide new active compounds exhibiting anti-proliferative activity against human cancer cell lines as well as leucyl aminopeptidase inhibitory properties, which could be used as therapeutic agents or pharmaceutical preparations in cancer treatment. A further aim of the invention is to provide a convenient method of chemical synthesis of such compounds.

Unexpectedly, this objective has been achieved with new 3,4-dihydroisoquinoline derivatives, exhibiting the above-mentioned properties.

Hence, the object of the invention is a 3,4-dihydroisoquinoline derivative of structure represented by formula I: 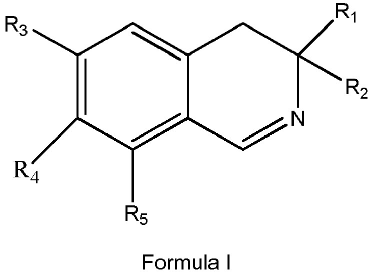 wherein
R₁ is selected from the group consisting of H, COOEt, COOH, COOBn
R₂ is selected from the group consisting of H, COOEt, COOH, COOBn
R₃ is selected from the group consisting of OH, OBn
R₄ is selected from the group consisting of OH, H, OBn
R₅ is selected from the group consisting of OH, OBn, wherein Bn is benzyl and Et is ethyl;
with the proviso that if each of R₁, R₂ and R₄ is H then at least one of R₃ and R₅ is OH, and isomers, salts, hydrates and solvates thereof.

Preferably, the compound according to the present invention is selected from 6,8-dihydroxy-3,4-dihydroisoquinoline-3,3-dicarboxylic acid diethyl ester 6,8-dibenzyloxy-3,4-dihydroisoquinoline-3,3-dicarboxylic acid diethyl ester 6,7,8-tribenzyloxy-3,4-dihydroisoquinoline-3,3-dicarboxylic acid dibenzyl ester 6,7,8-trihydroxy-3,4- dihydroisoquinoline-3-carboxylic acid and isomers, salts, hydrates and solvates thereof.

The object of the invention is also a method of preparation of a 3,4-dihydroisoquinoline derivative of structure represented by formula I: 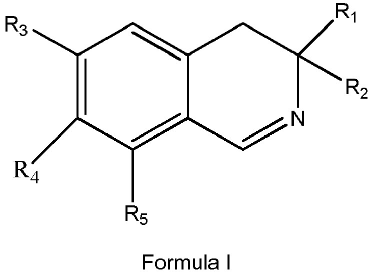 wherein
R₁ is selected from the group consisting of H, COOEt, COOH, COOBn
R₂ is selected from the group consisting of H, COOEt, COOH, COOBn
R₃ is selected from the group consisting of OH, OBn
R₄ is selected from the group consisting of OH, H, OBn
R₅ is selected from the group consisting of OH, OBn,
characterized by the fact that it involves steps, wherein
a) the compound of formula II 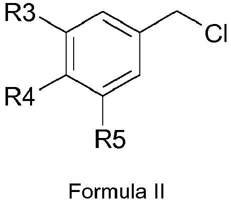 wherein
   R₃ is OBn
   R₄ is selected from the group consisting of H or OBn
   R₅ is OBn,
   and the remaining substituents are as defined above
   is reacted with diethyl or dibenzyl formamide malonate, yielding a compound of formula III 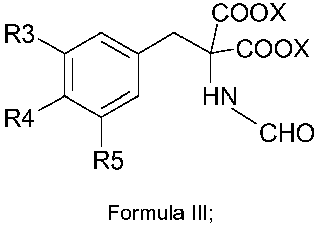 wherein
   R₃ is OBn
   R₄ is selected from the group consisting of H or OBn
   R₅ is OBn
   X is selected from the group consisting of Et or Bn;
   and the remaining substituents are as defined above
b) the compound of formula III is subjected to the Bischler-Napieralski cyclization reaction yielding a compound of formula IV 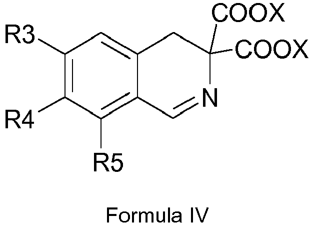 wherein
   R₃ is OBn
   R₄ is selected from the group consisting of H or OBn
   R₅ is OBn
   X is selected from the group consisting of Et or Bn;
   and the remaining substituents are as defined above;
   and optionally
c) the compound of formula IV is subjected to removal of benzyl groups, yielding a compound of formula I 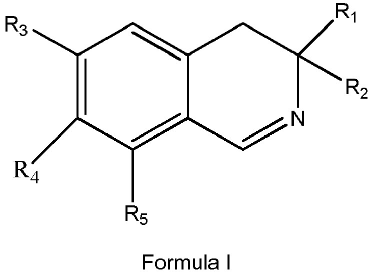 wherein
   R₁ is selected from the group consisting of COOEt, COOBn
   R₂ is selected from the group consisting of COOEt, COOBn
   R₃ is OH
   R₄ is selected from the group consisting of OH, H
   R₅ is OH
   or
c1) the compound of formula IV is subjected to removal of benzyl groups under acidic conditions, yielding a compound of formula I 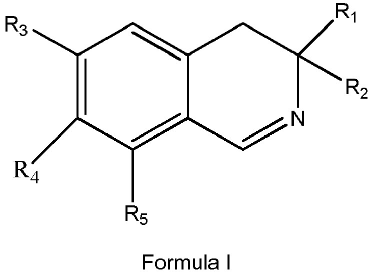 wherein
   R₁ is selected from the group consisting of H, COOH
   R₂ is selected from the group consisting of H, COOH
   R₃ is OH
   R₄ is selected from the group consisting of OH, H
   R₅ is OH.

Preferably, the method is characterized by the fact that in step c) and c1) boron tribromide is used.

The further object of the invention is a 3,4-dihydroisoquinoline derivative of structure represented by formula I: 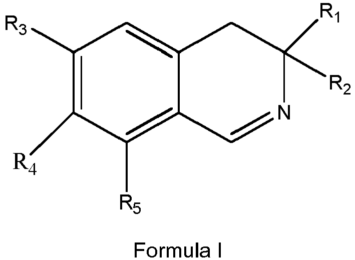 wherein the substituents are as defined above, as well as isomers, salts, hydrates and solvates thereof, for use as a medicament, in particular for use in the prevention, treatment and amelioration of symptoms of conditions associated with excessive activity of leucyl aminopeptidase and the conditions associated with excessive cell proliferation, as the conditions being cancer, in particular prostate cancer, colorectal cancer or leukemia.

The invention also relates to a pharmaceutical composition, characterized by the fact that it comprises a compound according to the present invention as an active agent.

The object of the invention is also the above-mentioned pharmaceutical composition for use as a medicament, in particular for use in the prevention, treatment and amelioration of symptoms of conditions associated with excessive activity of leucyl aminopeptidase and the conditions associated with excessive cell proliferation, as the conditions being cancer, in particular prostate cancer, colorectal cancer or leukemia.

The composition according to the present invention is characterized by the fact that it is in liquid form, for example for oral, intranasal, rectal, intravaginal, intragastric administration in the form of a solution, syrup or elixir; and for parenteral, subcutaneous, intradermal, intramuscular or intravenous administration (as via injection) in the form of a sterile solution or emulsion, or in solid form, such as a tablet, pill, capsule, granule, coated tablet and powder.

The further object of the invention is the use of the compound according to the present invention for use in the manufacture of a medicament for use in the prevention, treatment and amelioration of symptoms of conditions associated with excessive activity of leucyl aminopeptidase and the conditions associated with excessive cell proliferation, as the conditions being cancer, in particular prostate cancer, colorectal cancer or leukemia, preferably the use, wherein the compound according to the invention is used for the manufacture of a medicament in liquid form, such as a solution, syrup, elixir, sterile solution or emulsion, or in solid form, such as a tablet, pill, capsule, granule, coated tablet and powder.

The new 3,4-dihydroisoquinoline derivatives according to the present invention exhibit anti-proliferative activity against human cancer cell lines as well as leucyl aminopeptidase inhibitory properties. Therefore they may be used as therapeutic agents or pharmaceutical preparations in the treatment of cancer, as well as leucyl aminopeptidase inhibitors.

Due to the above properties, the compounds according to the invention may be used as therapeutic agents, in the form of pharmaceutical preparations, in particular in the treatment of cancer. The compounds according to the present invention exhibit a substantially strong activity against prostate cancer, colorectal cancer and leukemia. Therefore, they may be applied in a treatment for these cancer types.

The 3,4-dihydroisoquinoline-3-carboxylic acid derivatives were obtained in a several-step synthesis, where the key steps were the alkylation reaction with the use of benzyl or ethyl formamide malonate and the Bischler-Napieralski cyclization (Bischler, A.; Napieralski, B.: A new method for the synthesis of isoquinolines Chem. Ber. 1893, 26, 1903-1908). The use of boron tribromide in the last step allowed for the removal of all protecting groups and shortening the synthesis. The products were purified with standard methods.

The chemical synthesis of the compounds according to the invention, as well as their physicochemical properties and biological assays are provided in the examples.

### Examples

### Example 1

### Preparation of 6,8-dihydroxy-3,4-dihydroisoquinoline-3,3-dicarboxylic acid diethyl ester

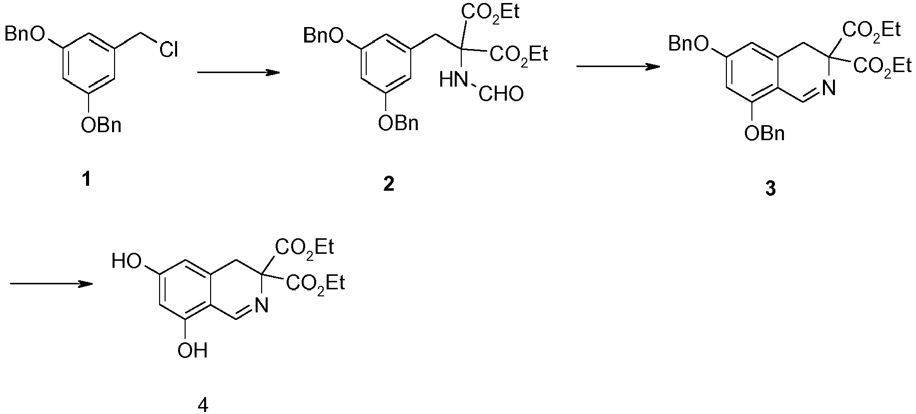

### 1.1. 3,5-dibenzyloxybenzyl-2-(formylamide)propanedioic acid diethyl ester 2

Diethyl formamide malonate (20.8 g, 100 mmol) was added to the solution of 3,5-dibenzyloxybenzyl chloride (34 g, 100 mmol) in acetone (500 ml), followed by K₂CO₃ (186 g, 1.333 mol) and Kl (49.9 g, 301 mmol). The reaction was conducted under anhydrous conditions, with vigorous stirring and heating (bath temperature 60°C) under reflux condenser for 35 hours. After this time the mixture was cooled and filtered through Celite. Subsequently, the solvent was evaporated and the product crystallized from hexane. 47.5 g of the product (94%) was obtained.
¹H NMR (CDCl₃) δ: 1.27 (t, J=7.1Hz, 6H); 3.56 (s, 2H); 4.14-4.32 (m, 4H); 5.0 (s, 4H); 6.24 (d, J=2.2 Hz, 2H); 6.53 (t, J=2.3Hz, 1H); 6.56 (bs, 1H); 7.28-7.44 (m, 10H); 7.92 (d, J=0.8Hz, 1H).
¹³C NMR (CDCl₃) δ: 13.9; 38.0; 62.8; 66.5; 69.9; 101.1; 109.2; 127.3; 127.9; 128.6; 136.8; 136.9; 159.7; 159.8; 166.9.
HR MS ESI calculated for C₂₉H₃₁NO₇ (M+H) 506.2173. Measured 506.2164.

### 1.2. 6,8-dibenzyloxy-3,4-dihydroisoquinoline-3,3-dicarboxylic acid diethyl ester 3

POCl₃ (0.45 ml) was added to the solution of 3,5-dibenzyloxybenzyl-2-(formylamide) propanedioic acid diethyl ester (0.8 g, 1.58 mmmol) in acetonitrile (40 ml). The reaction was conducted under argon atmosphere, with stirring and heating (bath temperature 75°C) under reflux condenser for 48 hours. After this time the solvent was evaporated on a rotary evaporator and a mixture of NaHCO₃ (20 ml) and CH₂Cl₂ (20 ml) was added. The mixture was extracted with CH₂Cl₂ (3 x 15 ml). The organic layers were combined and dried with anhydrous MgSO₄. After separation from the drying agent the solvent was evaporated. The product was crystallized from a mixture of AcOEt : hexane, (1:1). 0.64 g (83%) was obtained.
¹H NMR (CDCl₃) δ: 1.24 (t, J=7.1Hz, 6H); 3.3 (s, 2H); 4.13-4.32 (m, 4H); 5.0 (s, 2H); 5.1 (s, 2H); 6.41 (d, J=1.6 Hz, 1H); 6.44 (d, J=2.0Hz, 1H); 7.29-7.46 (m, 10H); 8.86 (d, J=0.5Hz, 1H).
¹³C NMR (CDCl₃) δ: 13.9; 31.5; 62.1; 69.8; 70.2; 99.1; 105.8; 111.4; 127.0-128.7; 136.1; 136.2; 136.9; 157.4; 158.1; 162.8; 169.3.
HR MS ESI calculated for C₂₉H₃₀NO₆ (M+H) 488.2068. Measured 488.2060.

### 1.3. 6,8-dihydroxy-3,4-dihydroisoquinoline-3,3-dicarboxylic acid diethyl ester 4

BBr₃ (0.05 ml, 0.54 mmol) was added dropwise to the solution of 6,8-dibenzyloxy-3,4-dihydroisoquinoline-3,3-dicarboxylic acid diethyl ester 3 (120 mg, 0.25 mmol) in CH₂Cl₂ (20 ml) at -70°C. The reaction was conducted under argon atmosphere at -70°C for 0.5 hour. After this time H₂O (20 ml) was added to the mixture and the whole mixture was stirred for 0.5 hour. Next, the mixture was washed 3 times with CH₂Cl₂. The obtained product was purified on Biogel P-2, eluting with 0.1% TFA solution in H₂O. 63 mg (83%) of the product was obtained.
¹H NMR (D₂O) δ: 1.26 (t, J=7.1Hz, 6H); 3.65 (s, 2H); 4.34 (q, J=7.1Hz, 4H); 6.32 (d, J=2.1Hz, 1H); 6.48 (dt, J=2.1Hz, 1.0Hz, 1H); 8.9 (d, J=0.8Hz, 1H).
¹³C NMR (D₂O) δ: 12.9; 31.3; 65.1; 66.1; 101.3; 105.2; 109.9; 138.0; 158.7; 165.3; 165.9; 170.1.
HR MS ESI calculated for (M+H) C₁₅H₁₈NO₆ 308.1129. Measured: 308.1120.

### Example 2

### Preparation of 6,7,8-trihydroxy-3,4-dihydroisoquinoline-3-carboxylic acid

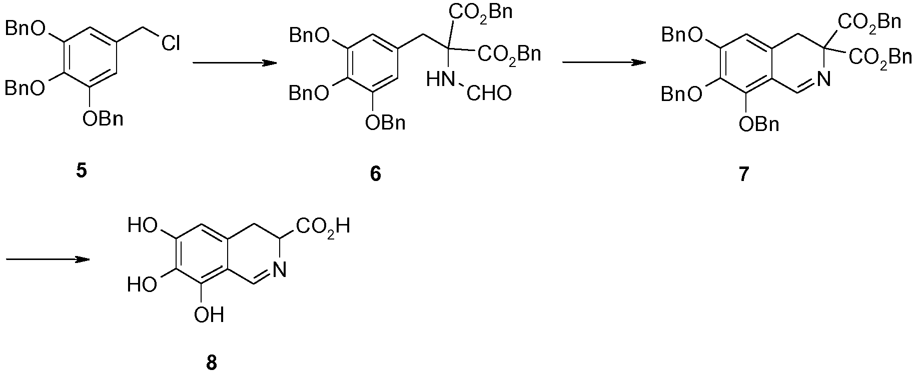

### 2.1. 3,4,5-tribenzyloxybenzyl-2-(formylamide) propanedioic acid dibenzyl ester 6

Dibenzyl formamide malonate (7.4 g; 22.02 mmol) was added to the solution of 3,4,5-tribenzyloxybenzyl chloride 5 (10 g, 22.02 mmol) in acetone (100 ml), followed by K₂CO₃ (41 g, 293 mmol) and Kl (10 g, 22.02 mmol). The reaction was conducted under anhydrous conditions, with vigorous stirring and heating (bath temperature 60°C) under reflux condenser for 24 hours. After this time the mixture was cooled and filtered through Celite. Next, the solvent was evaporated and the product was crystallized from hexane. 13.87 g of the product (86%) was obtained.
¹H NMR (CDCl₃) δ: 3.54 (s, 2H); 4.96 (s, 4H); 5.04 (s, 4H); 5.10 (s, 2H); 6.20 (s, 2H); 6.37 (bs, 1H); 7.16-7.46 (m, 25H); 7.72 (d, J=1.2Hz, 1H).
¹³C NMR (CDCl₃) δ: 38.0; 66.7; 68.1; 70.9; 75.0; 109.8; 127.1-128.6; 129.9; 134.5; 137.1; 137.7; 152.4; 159.8; 166.5.
HR MS ESI calculated for C₄₆H₄₂NO₈ (M+H) 736.2905. Measured 736.2845.

### 2.2. 6,7,8-tribenzyloxy-3,4-dihydroisoquinoline-3,3-dicarboxylic acid dibenzyl ester 7

POCl₃ (2.52 ml) was added to the solution of 3,4,5-tribenzyloxybenzyl-2-(formylamide) propanedioic acid dibenzyl ester (6.48 g, 8.81 mmol) in acetonitrile (70 ml). The reaction was conducted under argon atmosphere, with stirring and heating (bath temperature 75°C) under reflux condenser for 24 hours. After this time the solvent was evaporated on a rotary evaporator and a mixture of NaHCO₃ (20 ml) and CH₂Cl₂ (20 ml) was added. The mixture was extracted with CH₂Cl₂ (3 x 15 ml). The organic layers were combined and dried with anhydrous MgSO₄. After separation from the drying agent the solvent was evaporated. The product was crystallized from a mixture of AcOEt : hexane, (1:3). 5.6 g (89%) was obtained.
¹H NMR (CDCl₃) δ: 3.27 (s, 2H); 5.01 (s, 2H); 5.03 (s, 2H); 5.08 (s, 2H); 5.10 and 5.19 (AB, J=12Hz, 4H); 6.53 (s, 1H); 7.17-7.44 (m, 25H); 8.73 (s, 1H).
¹³C NMR (CDCl₃) δ: 29.7; 31.1; 67.6; 70.4; 70.9; 75.6; 108.3; 115.5; 127.4-128.7; 130.1; 135.2; 136.0; 136.6; 137.1; 140.3; 151.7; 156.3; 158.2; 168.9.
HR MS ESI calculated for C₄₆H₄₀NO₇ (M+H) 718.2799. Measured 718.2793.

### 2.3. 6,7,8-trihydroxy-3,4-dihydroisoquinoline-3-carboxylic acid 8

BBr₃ (1.75 ml, 17.97 mmol) was added dropwise to the solution of 6,7,8-tribenzyloxy-3,4-dihydroisoquinoline-3,3-dicarboxylic acid dibenzyl ester (2.15 g, 3.0 mmol) in CH₂Cl₂ (60 ml) at -20°C. The reaction was conducted under argon atmosphere at -20°C for 0.5 hour. After this time a solution of 10% HCI (20 ml) was added to the mixture and the whole mixture was stirred for 0.5 hour. Next, the mixture was washed 3 times with CH₂Cl₂. The product was crystallized from H₂O. 0.6 g of the product (90%) was obtained.
¹H NMR (D₂O) δ: 3.00 and 3.10 (ABX, J= 0.7Hz, 8.6Hz, 17.2Hz, 2H); 4.59 (ddd, J=8.6Hz, 7.3Hz, 1.2Hz, 2H); 6.21 (s, 1H); 8.6 (s, 1H).
¹³C NMR (D₂O) δ: 27.3; 53.2; 105.8; 108.7; 129.8; 130.5; 151.6; 156.8; 159.5; 171.0.
HR MS ESI calculated for (M+H) C₁₀H₁₀NO₅ 224.0559. Measured: 224.0594.

### Example 3

**Leucyl aminopeptidase inhibition** according to Cahan R., Hetzroni E., Nisnevitch M., Nitzan Y.: Purification and Identification of a Novel Leucine Aminopeptidase from Bacillus Thuringiensis israelensis. Curr. Microbial (2007) 55: 413-419.

### Reagents:

1. Enzyme: pig kidney microsomal leucyl aminopeptidase, Type IV-S (Sigma-Aldrich) 3.5 mg of protein/ml, 28 U/mg of protein; diluted with 0.02M Tris-HCl + 0.5 mM CaCl₂ buffer pH=8.5 until activity 0.01225U achieved
2. Substrate: L-leucine p-nitroanilide: 4.2 mg / 269 µl DMSO; solution diluted 1:19 with 0.02 M Tris-HCl buffer, pH = 8.5 + 0.5 mM CaCl₂
3. 0.02 M Tris-HCl buffer, pH = 8.5 + 0.5 mM CaCl₂
4. DMSO, inhibitor solution in DMSO

### Assay procedure:

5 µl of leucyl aminopeptidase (0.245 U), 70 µl of Tris, 5 µl of inhibitor solution in different concentrations (in DMSO) were added to the wells of a microtitration plate, followed by pre-incubation for 30 minutes at 37 °C. Next, 20µl of diluted L-leucine p-nitroanilide solution was added, whereupon the reaction mixture was incubated for 20 minutes at 37 °C, with measurement of absorbance changes for wavelength 405 nm (Fluorostar, Omega, BMG Labtech). The control sample for the enzyme contained analogous components, while instead of the inhibitor solution DMSO (5 µl) was added.

For each inhibitor concentration the percentage inhibition was calculated. The inhibitor concentration required for 50% of enzyme activity inhibition (IC₅₀) was calculated based on linear regression data between the percentage inhibition and the concentration for a given inhibitor.

IC₅₀ for compound **3** is 0.028 mM, for **4** it is 1.35 mM; for **8** it is 3.8 mM.

### Example 4

4.1. Anti-proliferative activity evaluation using the SRB test [Skehan et al., J. Natl. Cancer Inst., 82: 1107-1112, 1990] against human cancer cell lines with diverse tissue origins and determination of the IC₅₀ concentration:
a) colorectal cancer LoVo,
b) colorectal cancer LoVo/DX (with multidrug resistance),
c) breast cancer MCF-7,
d) prostate cancer LNCaP,
e) bladder cancer HCV29T,
f) kidney cancer A498,
g) lung cancer A549

4.2. Anti-proliferative activity evaluation using the MTT test [Marcinkowska et al.: J. Steroid Biochem. Mol. 76: 71-78, 1998] against human cancer cell lines with diverse tissue origins and determination of the IC₅₀ concentration:
a) promyelocytic leukemia HL-60,
b) promyelocytic leukemia HL-60/MX2 (with multidrug resistance)

### 4.3. Cytotoxic activity evaluation against normal cell lines with diverse tissue origins using the SRB test:

a) normal mouse fibroblasts (BALB/3T3),
b) normal mammary gland cells (MCF-10),
c) normal human epithelium (HLMEC)

### Preparations

The stock solution of the tested compound with concentration of 20 mg/ml was prepared day before the analysis by dissolving the preparation in DMSO, followed by storage in -20°C. After 24 hours the preparation was thawed and diluted with the culture medium. The compounds were tested in concentrations of 100, 10, 1, 0.1 0.01 µg/ml.

### Cell lines

The following human cancer cell lines were used in the study: HL-60 (promyelocytic leukemia), HL-60/MX2 (promyelocytic leukemia, line resistant to mitoxantrone), MCF7 (mammary gland cancer), LoVo (colorectal cancer), LoVo/Dx (colorectal cancer with multidrug resistance), HCV29T (bladder cancer), A498 (kidney cancer), A549 (lung cancer) and LNCaP (prostate cancer), as well as normal cell lines: BALB3T3 (normal mouse fibroblasts), MCF-10 (normal mammary gland cells) and HLMEC (normal human endothelium).

The lines are in the cell line bank of The Polish Academy of Sciences Institute of Immunology and Experimental Therapy (Instytut Immunologii i Terapii Doswiadczalnej PAN) (obtained from the ATCC collection). The composition of culture media is summarized in Table 1. All media contained antibiotics: 100 µg/ml streptomycin and 100 U/ml penicillin. The cells were cultured in humidified atmosphere 5% CO₂ at 37°C.

**Table 1**

| The composition of culture media | | | |
|---|---|---|---|
| No. | Cell line | Medium | Supplements |
| 1 | BALB3T3 | Dulbecco | 10% FBS, L-glutamine [2 mM] |
| 2 | MCF-10A | Ham's F12 Nutrient Mix with glutamine | 5% HS (Gibco), Insulin [0.01 mg/ml], EGF [20 ng/ml], hydrocortisone [0.5 µg/ml], cholera toxin [0.05 µg/ml] |
| 3 | HLMEC | RPMI-1640 | 10% FBS (HyClone), L-glutamine [2 mM] |
| 4 | HL60, HL60MX2 | RPMI-1640 | 10% FBS, L-glutamine [4 mM], sodium pyruvate [1 mM], glucose [4.5 g/l] |
| 5 | MCF7 | Eagle | 10% FBS, L-glutamine [2 mM], amino acids, insulin [0.01 mg/ml] |
| 6 | Lovo | OptiMEM +RPMI(1:1) | 5% FBS (HyClone), L-glutamine [2 mM], sodium pyruvate [1 mM] |
| 7 | Lovo/Dx | OptiMEM +RPMI(1:1) | 5% FBS (HyClone), L-glutamine [2 mM], sodium pyruvate [1 mM], doxorubicin [10 ug/100 ml] |
| 8 | HCV29T | OptiMEM +RPMI(1:1) | 5% FBS(HyClone), L-glutamine [2 mM] |
| 9 | A498 | OptiMEM +RPMI(1:1) + G.MAX | 5% FBS (HyClone), sodium pyruvate [1 mM] |
| 10 | A549 | OptiMEM +RPMI(1:1) | 5% FBS (HyClone), L-glutamine [2 mM] |
| 11 | LNCaP | OptiMEM +RPMI(1:1) + G.MAX | 8% FBS (HyClone) sodium pyruvate [0.5 mM], glucose [1%] |

### Cytotoxicity assays

The cells in the phase of logarithmic growth were seeded on a 96-well plate (Sarsted) in quantity of 1 x 10⁴ cells/well, and then incubated at 37°C, 5% CO₂ for 24 hours. Subsequently, the tested compound was added in different concentrations and the incubation was carried out. The assays measured the inhibition of target cells proliferation in a 72-hour culture *in vitro.* For each assay the samples containing predetermined preparation concentrations were applied in triplicates. The experiments were repeated 3-5 times.

### The MTT method

The method involves a measurement of cell number increase inhibition utilizing the redox activity of mitochondria. The determination of biological activity with the MTT (3-(4,5-dimethylthiazol-2-yl)-2,5-diphenyltetrazolium bromide) assay is based on the assumption that reduction of the dye occurs only in viable cells. As a result of mitochondrial dehydrogenase activity the water-soluble tetrazolium salt (thiazolyl blue formazan - MTT) is converted to water-insoluble formazan crystals. The quantity of the formed formazan crystals is proportional to the number of cells.

3-4 hours before the end of cell incubation 20 µl of MTT solution (5 mg/ml) was added to each well. Next, 80 µl of dissolving mixture (with the following composition: 225 ml DMF (POCh), 67.5 g SDS (Sigma-Aldrich), 275 ml distilled H₂O) was added. After 24 hours, formazan crystals were dissolved and OD of the samples was measured for wavelength 570 nm (Multiskan PC photometer, Labsystem, Helsinki).

### The SRB method

Sulphorhodamine B (SRB) is an anionic dye which binds to essential amino acids of cellular proteins. The determination of cytotoxic activity in the assay is based on the measurement of the amount of cellular proteins.

After the incubation is finished the attached cells are fixed with cold 50% TCA (4°C) at 4°C for 1 hour. Next, each well is washed with water and air dried, repeating this step 5 times.

0.4% SRB solution (Sigma-Aldrich) dissolved in 1% acetic acid is added consecutively to each well and the staining is carried out for 30 minutes. The unbound dye is removed and the cells are washed 4 times with 1% acetic acid. Next, the plate is air dried for about 5 minutes.

The bound dye is dissolved by adding 10 mM Tris-base to each well and then OD is determined for wavelength 540 nm (Multiscan RC photometer, Labsystes, Helsinki).

### Results

The results of the assays in the form of IC₅₀ (the concentration causing proliferation inhibition of 50% of a cancer cells population) determined for the tested compounds and cisplatin are summarized in Table 2 [Geran RI et al.: Cancer Chemotherapy Reports, 3, 2 (part3): 59-61, 1972].

DMSO, which is a solvent for the compound, caused proliferation inhibition only in the highest concentration: MCF-7 - 31 %, Balb3T3 - 23%, Lovo - 62%, Lovo/Dx - 0%, HCV29T - 17%, A549 - 8%, A498 - 29% and LNCaP - 37% respectively. Since the IC₅₀ values of the tested compound are lower than 100 µg/ml this did not influence the arithmetically calculated IC₅₀ value.

**Table 2**

| Anti-proliferative activity expressed as IC₅₀ value for a compound against cancer cells and cytotoxic activity against normal cells | | | |
|---|---|---|---|
| Cell line | **Compound 3 IC₅₀ (µg/ml)** | **Compound 8 IC₅₀ (µg/ml)** | **Cisplatin IC₅₀ (µg/ml)** |
| Balb/3T3 (normal mouse fibroblasts) | 17.36 +/- 7.9 | 52.5 +/- 22.3 | 2.4 +/- 0.3 |
| MCF-10 (normal mammary gland cells) | 15.3 | 18.9 +/- 11.4 | 0.7 |
| HLMEC (normal human epithelium) | 3.3 +/- 0.3 | 33.4 +/- 5.0 | 2.6+/-0.3 |
| HL-60 (promyelocytic leukemia) | 0.12 | 27.7 +/- 4.3 | 0.4 +/- 0.1 |
| HL-60 MX2 (promyelocytic leukemia with multidrug resistance) | 1.3 | 33.2 +/- 2.7 | 0.32 +/- 0.02 |
| MCF-7 (breast cancer) | 4.7 +/- 0.8 | 18.9 +/- 11.4 | 3.6 +/- 0.6 |
| Lovo (colorectal cancer) | 3.1 +/- 0.3 | 33.6 +/- 9.5 | 2.5 +/- 1.3 |
| Lovo/Dx (colorectal cancer with multidrug resistance) | 2.0 +/- 0.8 | 30.1 +/- 2.3 | 3.0 +/- 0.7 |
| LNCaP (prostate cancer) | 1.6 +/- 0.3 | 30.9 +/-5.7 | 6.2 +/- 1.3 |
| HCV29T (bladder cancer) | 4.3 +/- 0.7 | 28.7 +/- 7.2 | 2.7 +/- 0.6 |
| A549 (lung cancer) | 6.4 +/- 2.3 | 44.3 +/- 6.7 | 3.2 +/- 1.0 |
| A498 (kidney cancer) | 6.5 +/- 1.5 | 40.0 +/- 18.4 | 2.8 +/- 0.6 |

## Claims

1. 3,4-dihydroisoquinoline derivative of structure represented by formula I: 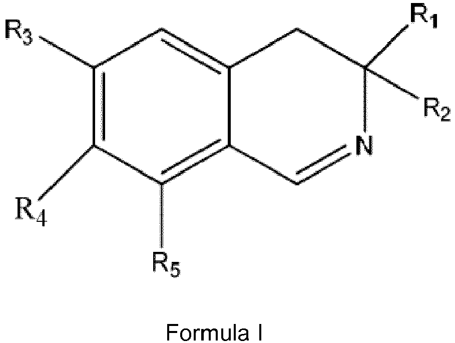 wherein
R₁ is selected from the group consisting of H, COOEt, COOH, COOBn
R₂ is selected from the group consisting of H, COOEt, COOH, COOBn
R₃ is selected from the group consisting of OH, OBn
R₄ is selected from the group consisting of OH, H, OBn
R₅ is selected from the group consisting of OH, OBn,
with the proviso that if each of R₁, R₂ and R₄ is H then at least one of R₃ and R₅ is OH,
and isomers, salts, hydrates and solvates thereof.

2. The compound according to claim 1, selected from:
6,8-dihydroxy-3,4-dihydroisoquinoline-3,3-dicarboxylic acid diethyl ester
6,8-dibenzyloxy-3,4-dihydroisoquinoline-3,3-dicarboxylic acid diethyl ester
6,7,8-tribenzyloxy-3,4-dihydroisoquinoline-3,3-dicarboxylic acid dibenzyl ester
6,7,8-trihydroxy-3,4- dihydroisoquinoline-3-carboxylic acid
and isomers, salts, hydrates and solvates thereof.

3. A method of preparation of 3,4-dihydroisoquinoline derivative of structure represented by formula I: 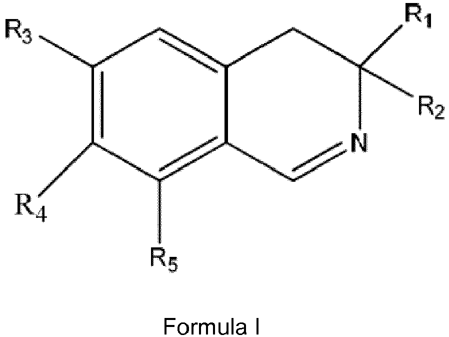 wherein
R₁ is selected from the group consisting of H, COOEt, COOH, COOBn
R₂ is selected from the group consisting of H, COOEt, COOH, COOBn
R₃ is selected from the group consisting of OH, OBn
R₄ is selected from the group consisting of OH, H, OBn
R₅ is selected from the group consisting of OH, OBn,
**characterized in that** it involves steps, wherein
a) the compound of formula II 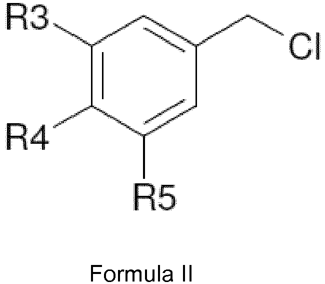 wherein
R₃ is OBn
R₄ is selected from the group consisting of H or OBn
R₅ is OBn,
and the remaining substituents are as defined above
is reacted with diethyl or dibenzyl formamide malonate, yielding a compound of formula III 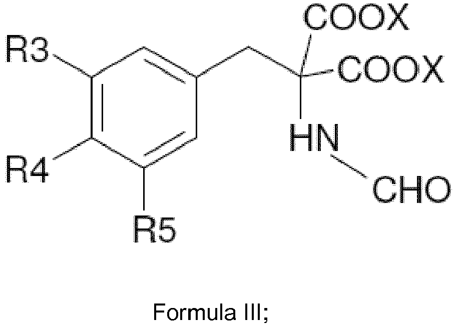 wherein
R₃ is OBn
R₄ is selected from the group consisting of H or OBn
R₅ is OBn
X is selected from the group consisting of Et or Bn;
and the remaining substituents are as defined above
b) the compound of formula III is subjected to the Bischler-Napieralski cyclization reaction yielding a compound of formula IV 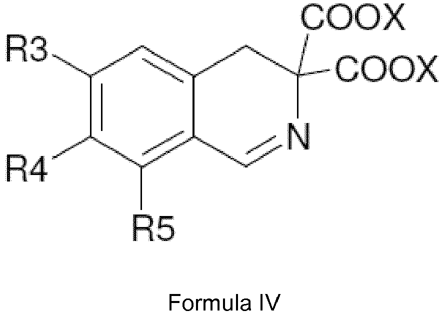 wherein
R₃ is OBn
R₄ is selected from the group consisting of H or OBn
R₅ is OBn
X is selected from the group consisting of Et or Bn;
and the remaining substituents are as defined above;
and optionally
c) the compound of formula IV is subjected to removal of benzyl groups, yielding a compound of formula I 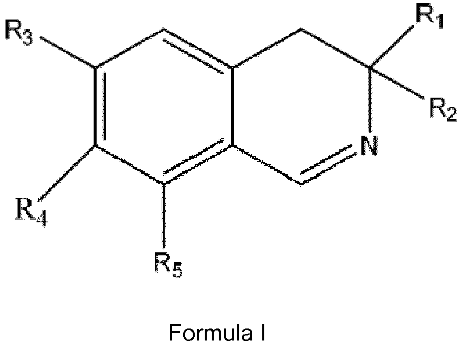 wherein
R₁ is selected from the group consisting of COOEt, COOBn
R₂ is selected from the group consisting of COOEt, COOBn
R₃ is OH
R₄ is selected from the group consisting of OH, H
R₅ is OH
or
c1) the compound of formula IV is subjected to removal of benzyl groups under acidic conditions, yielding a compound of formula I 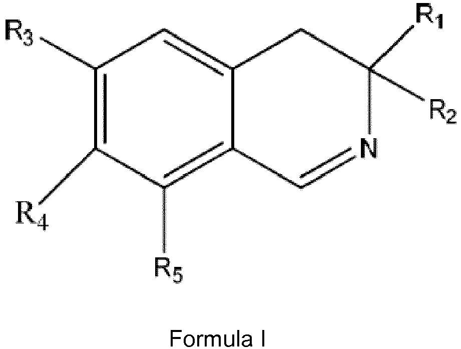 wherein
R₁ is selected from the group consisting of H, COOH
R₂ is selected from the group consisting of H, COOH
R₃ is OH
R₄ is selected from the group consisting of OH, H
R₅ is OH.

4. The method according to claim 3, **characterized in that** in step c) and c1) boron tribromide is used.

5. The compound according to claim 1 for use as a medicament.

6. The compound according to claim 1 for use in prevention, treatment and amelioration of symptoms of conditions associated with excessive activity of leucyl aminopeptidase and the conditions associated with excessive cell proliferation, the conditions being cancer, in particular prostate cancer, colorectal cancer or leukemia.

7. A pharmaceutical composition **characterized in that** it comprises the compound according to claim 1 as an active agent.

8. The composition according to claim 7 for use as a medicament.

9. The composition according to claim 7 or 8 for use in prevention, treatment and amelioration of symptoms of conditions associated with excessive activity of leucyl aminopeptidase and the conditions associated with excessive cell proliferation, the conditions being cancer, in particular prostate cancer, colorectal cancer or leukemia.

10. The composition according to any of claims 7-9, **characterized in that** it is in liquid form, for example for oral, intranasal, rectal, intravaginal, intragastric administration in the form of a solution, syrup or elixir; and for parenteral, subcutaneous, intradermal, intramuscular or intravenous administration (as via injection) in the form of a sterile solution or emulsion, as well as in solid form, such as a tablet, pill, capsule, granule, coated tablet and powder.

11. Use of the compound according to claim 1 in the manufacture of a medicament for use in prevention, treatment and amelioration of symptoms of conditions associated with excessive activity of leucyl aminopeptidase and the conditions associated with excessive cell proliferation, such as cancer, in particular prostate cancer, colorectal cancer or leukemia.

12. The use according to claim 11, wherein the compound according to claim 1 is used in the manufacture of a medicament in liquid form, such as a solution, syrup, elixir, sterile solution or emulsion, or in solid form, such as a tablet, pill, capsule, granule, coated tablet and powder.

## Patentansprüche

1. 3,4-Dihydroisochinolin-Derivat mit der durch die Formel I veranschaulichten Struktur: 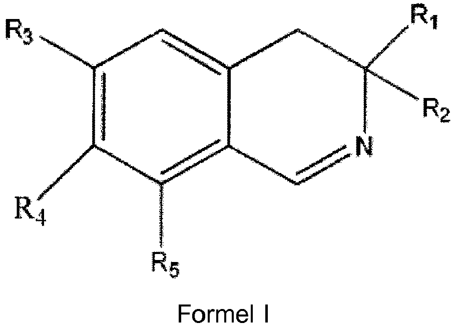 wobei
R₁ ausgewählt ist aus der Gruppe bestehend aus H, COOEt, COOH, COOBn
R₂ ausgewählt ist aus der Gruppe bestehend aus H, COOEt, COOH, COOBn
R₃ ausgewählt ist aus der Gruppe bestehend aus OH, OBn
R₄ ausgewählt ist aus der Gruppe bestehend aus OH, H, OBn
R₅ ausgewählt ist aus der Gruppe bestehend aus OH, OBn,
mit der Maßgabe, dass, wenn sowohl R₁, R₂ als auch R₄ H sind, mindestens eines von R₃ und R₅ OH sind,
und Isomere, Salze, Hydrate und Solvate davon.

2. Verbindung nach Anspruch 1, ausgewählt aus:
6,8-Dihydroxy-3,4-dihydroisochinolin-3,3-dicarbonsäurediethylester
6,8-Dibenzyloxy-3,4-dihydroisochinolin-3,3-dicarbonsäurediethylester
6,7,8-Tribenzyloxy-3,4-dihydroisochinolin-3,3-dicarbonsäuredibenzylester
6,7,8-Trihydroxy-3,4-dihydroisochinolin-3-carbonsäure
und Isomere, Salze, Hydrate und Solvate davon.

3. Verfahren zur Herstellung eines 3,4-Dihydroisochinolin-Derivats mit der durch die Formel I veranschaulichten Struktur: 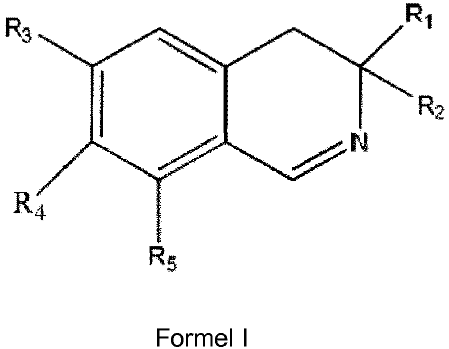 wobei
R₁ ausgewählt ist aus der Gruppe bestehend aus H, COOEt, COOH, COOBn
R₂ ausgewählt ist aus der Gruppe bestehend aus H, COOEt, COOH, COOBn
R₃ ausgewählt ist aus der Gruppe bestehend aus OH, OBn
R₄ ausgewählt ist aus der Gruppe bestehend aus OH, H, OBn
R₅ ausgewählt ist aus der Gruppe bestehend aus OH, OBn,
**dadurch gekennzeichnet, dass** es Schritte umfasst, wobei
a) die Verbindung der Formel II 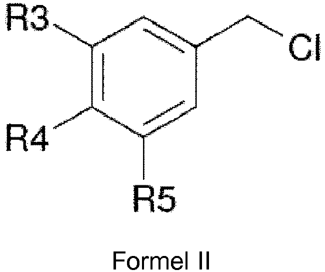 wobei
R₃ OBn ist
R₄ ausgewählt ist aus der Gruppe bestehend aus H oder OBn
R₅ OBn ist
und die übrigen Substituenten wie oben definiert sind
mit Diethyl- oder Dibenzylformamidmalonat umgesetzt wird, wodurch eine Verbindung der Formel III erhalten wird 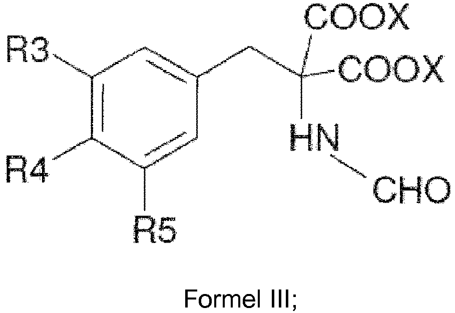 wobei
R₃ OBn ist
R₄ ausgewählt ist aus der Gruppe bestehend aus H oder OBn
R₅ OBn ist
X ausgewählt ist aus der Gruppe bestehend aus Et oder Bn;
und die übrigen Substituenten wie oben definiert sind
b) die Verbindung der Formel III der Bischler-Napieralski-Cyclisierungsreaktion unterzogen wird, wodurch eine Verbindung der Formel IV erhalten wird 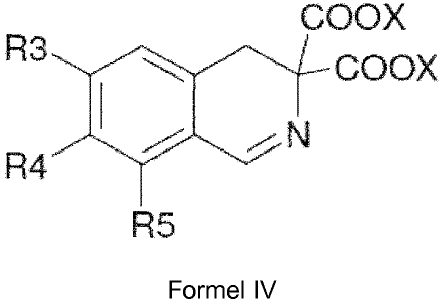 wobei
R₃ OBn ist
R₄ ausgewählt ist aus der Gruppe bestehend aus H oder OBn
R₅ OBn ist
X ausgewählt ist aus der Gruppe bestehend aus Et oder Bn;
und die übrigen Substituenten wie oben definiert sind;
und gegebenenfalls
c) die Verbindung der Formel IV einer Entfernung von Benzylgruppen unterzogen wird, wodurch eine Verbindung der Formel I erhalten wird 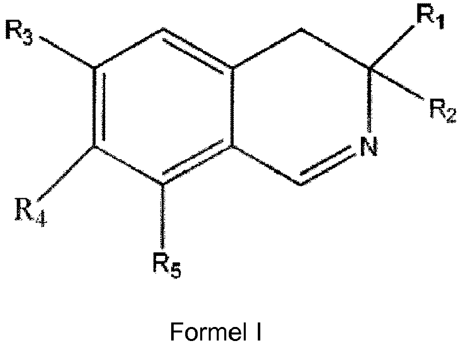 wobei
R₁ ausgewählt ist aus der Gruppe bestehend aus COOEt, COOBn
R₂ ausgewählt ist aus der Gruppe bestehend aus COOEt, COOBn
R₃ OH ist
R₄ ausgewählt ist aus der Gruppe bestehend aus OH, H
R₅ OH ist
oder
c1) die Verbindung der Formel IV einer Entfernung von Benzylgruppen unter sauren Bedingungen unterzogen wird, wodurch eine Verbindung der Formel I erhalten wird 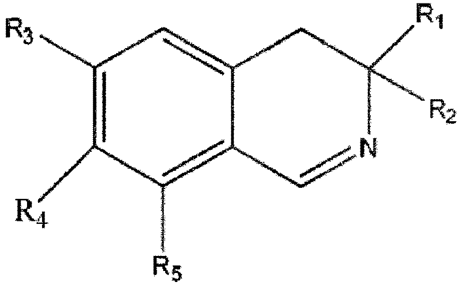 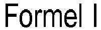 wobei
R₁ ausgewählt ist aus der Gruppe bestehend aus H, COOH
R₂ ausgewählt ist aus der Gruppe bestehend aus H, COOH
R₃ OH ist
R₄ ausgewählt ist aus der Gruppe bestehend aus OH, H
R₅ OH ist.

4. Verfahren nach Anspruch 3, **dadurch gekennzeichnet, dass** in Schritt c) und c1) Bortribromid verwendet wird.

5. Verbindung nach Anspruch 1 zur Verwendung als Medikament.

6. Verbindung nach Anspruch 1 zur Verwendung bei der Prävention, Behandlung und Verbesserung von Symptomen von Krankheiten im Zusammenhang mit einer übermäßigen Aktivität von Leucin-Aminopeptidase und der Krankheiten im Zusammenhang mit einer übermäßigen Zellvermehrung, wobei es sich bei den Krankheiten um Krebs, insbesondere um ein Prostatakarzinom, ein kolorektales Karzinom oder Leukämie handelt.

7. Pharmazeutische Zusammensetzung, **dadurch gekennzeichnet, dass** sie die Verbindung nach Anspruch 1 als Wirkstoff umfasst.

8. Zusammensetzung nach Anspruch 7 zur Verwendung als Medikament.

9. Zusammensetzung nach Anspruch 7 oder 8 zur Verwendung bei der Prävention, Behandlung und Verbesserung von Symptomen von Krankheiten im Zusammenhang mit einer übermäßigen Aktivität von Leucin-Aminopeptidase und der Krankheiten im Zusammenhang mit einer übermäßigen Zellvermehrung, wobei es sich bei den Krankheiten um Krebs, insbesondere um ein Prostatakarzinom, ein kolorektales Karzinom oder Leukämie handelt.

10. Zusammensetzung nach einem der Ansprüche 7-9, **dadurch gekennzeichnet, dass** sie in flüssiger Form, beispielsweise für eine orale, intranasale, rektale, intravaginale, intragastrale Verabreichung, in Form einer Lösung, eines Sirups oder Elixiers; und für eine parenterale, subkutane, intradermale, intramuskuläre oder intravenöse Verabreichung (wie mittels einer Injektion) in Form einer sterilen Lösung oder Emulsion sowie in fester Form, wie als Tablette, Pille, Kapsel, Granalie, beschichtete Tablette und ein Pulver, vorliegt.

11. Verwendung der Verbindung nach Anspruch 1 zur Herstellung eines Medikaments zur Verwendung bei der Prävention, Behandlung und Verbesserung von Symptomen von Krankheiten im Zusammenhang mit einer übermäßigen Aktivität von Leucin-Aminopeptidase und der Krankheiten im Zusammenhang mit einer übermäßigen Zellvermehrung, wie Krebs, insbesondere ein Prostatakarzinom, ein kolorektales Karzinom oder Leukämie.

12. Verbindung nach Anspruch 11, wobei die Verbindung nach Anspruch 1 bei der Herstellung eines Medikaments in flüssiger Form, wie einer Lösung, eines Sirups, Elixiers, einer sterilen Lösung oder Emulsion, oder in fester Form, wie einer Tablette, Pille, Kapsel, Granalie, beschichteten Tablette und eines Pulvers, verwendet wird.

## Revendications

1. Dérivés de 3,4-dihydroisoquinoline de structure représentée par la formule I: 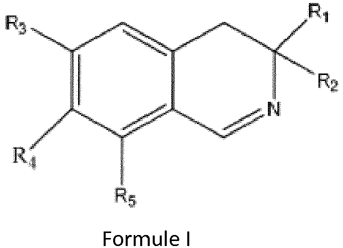 dans laquelle
R₁ est choisi parmi le groupe consistant en H, COOEt, COOH, COOBn
R₂ est choisi parmi le groupe consistant en H, COOEt, COOH, COOBn
R₃ est choisi parmi le groupe consistant en OH, OBn
R₄ est choisi parmi le groupe consistant en OH, H, OBn
R₅ est choisi parmi le groupe consistant en OH, OBn,
sous réserve que si chacun des R₁, R₂ et R₄ représente un H, alors, au moins un des groupes R₃ et R₅ est OH,
et leurs isomères, sels, hydrates et solvates.

2. Composé suivant la revendication 1, choisi parmi:
l'ester diéthylique de l'acide 6,8-dihydroxy-3,4-dihydroisoquinoline-3,3-dicarboxylique
l'ester diéthylique de l'acide 6,8-dibenzyloxy-3,4-dihydroisoquinoline-3,3-dicarboxylique
l'ester dibenzylique de l'acide 6,7,8-tribenzyloxy-3,4-dihydroisoquinoline-3,3-dicarboxylique
l'acide 6,7,8-trihydroxy-3,4-dihydroisoquinoline-3-carboxylique
et leurs isomères, sels, hydrates et solvates.

3. Méthode de préparation des dérivés de 3,4-dihydroisoquinoline de structure représentée par la formule I: 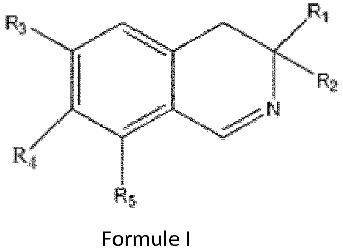 dans laquelle
R₁ est choisi parmi le groupe consistant en H, COOEt, COOH, COOBn
R₂ est choisi parmi le groupe consistant en H, COOEt, COOH, COOBn
R₃ est choisi parmi le groupe consistant en OH, OBn
R₄ est choisi parmi le groupe consistant en OH, H, OBn
R₅ est choisi parmi le groupe consistant en OH, OBn,
**caractérisé en ce qu'**elle comprend des étapes dans lesquelles,
a) on fait réagir le composé de formule II 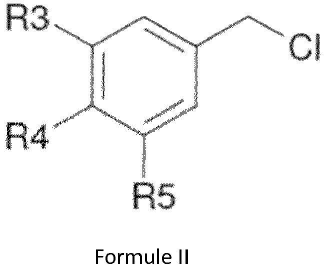 dans laquelle
R₃ représente OBn
R₄ est choisi parmi le groupe consistant en H ou OBn
R₅ représente OBn,
et les autres substituants sont tels que définis ci-dessus,
avec le malonate du diéthyl ou dibenzyl formamide, fournissant une composé deformule III 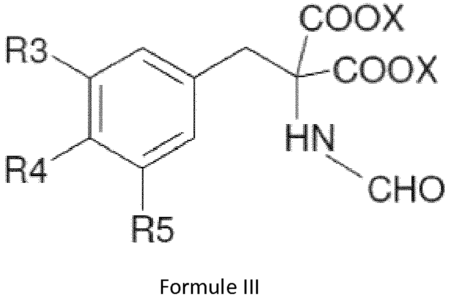 dans laquelle 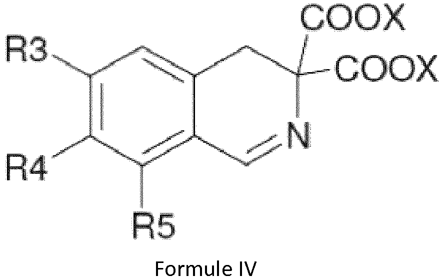
R₃ représente OBn
R₄ est choisi parmi le groupe consistant en H ou OBn
R₅ représente OBn
X est choisi parmi le groupe consistant en Et ou Bn;
et les autres substituants sont tels que définis ci-dessus
b) on soumet le composé de formule III à une réaction de cyclisation de Bischler-Napieralski fournissant un composé de formule IV 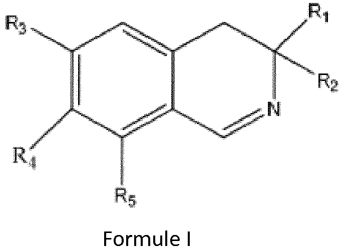 dans laquelle
R₃ représente OBn
R₄ est choisi parmi le groupe consistant en H ou OBn
R₅ représente OBn
X est choisi parmi le groupe consistant en Et ou Bn;
et les autres substituants sont tels que définis ci-dessus;
et optionnellement
c) on soumet le composé de formule IV à l'élimination des groupes benzyle, fournissant un composé de formule I
dans laquelle
R₁ est choisi parmi le groupe consistant en COOEt, COOBn
R₂ est choisi parmi le groupe consistant en COOEt, COOBn
R₃ représente OH
R₄ est choisi parmi le groupe consistant en OH, H
R₅ représente OH
ou
c1) le composé de formule IV est soumis à l'élimination des groupes benzyle sous conditions acide, fournissant un composé de formule I
dans laquelle
R₁ est choisi parmi le groupe consistant en H, COOH
R₂ est choisi parmi le groupe consistant en H, COOH
R₃ représente OH
R₄ est choisi parmi le groupe consistant en OH, H
R₅ représente OH.

4. Méthode suivant la revendication 3, **caractérisée en ce qu'**à l'étape c) et c1), on utilise le tribromure de bore.

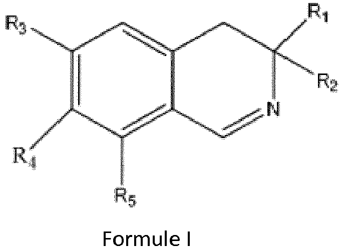

5. Composé suivant la revendication 1, pour utilisation en tant que médicament.

6. Composé suivant la revendication 1 pour utilisation dans la prévention, le traitement et l'amélioration des symptômes de conditions associé avec une activité excessive de la leucyl aminopeptidase et conditions associés avec une prolifération excessive de cellules, ces conditions étant le cancer, en particulier, le cancer de la prostate, le cancer colorectal ou la leucémie.

7. Composition pharmaceutique **caractérisée en ce qu'**elle comprend le composé suivant la revendication 1 en tant que principe actif.

8. Composition suivant la revendication 7 pour utilisation en tant que médicament.

9. Composition suivant la revendication 7 ou 8 pour utilisation dans la prévention, le traitement et l'amélioration des symptômes de conditions associé avec une activité excessive de la leucyl aminopeptidase et de conditions associés avec une prolifération excessive de cellules, ces conditions étant le cancer, en particulier, le cancer de la prostate, le cancer colorectal ou la leucémie.

10. Composition suivant l'une quelconque des revendications 7 à 9, **caractérisée en ce qu'**elle se présente sous forme liquide, par exemple pour administration orale, intranasale, rectale, intravaginale, intragastrique, sous forme d'une solution, d'un sirop ou élixir; et, pour administration parentérale, sous-cutanée, intradermique, intramusculaire ou intraveineuse (comme par injection) sous forme d'une solution ou émulsion stérile, ainsi que sous forme solide, telle que comprimé, pilule, capsule, granule, comprimé enrobé et poudre.

11. Utilisation du composé suivant la revendication 1 pour la fabrication d'un médicament pour utilisation dans la prévention, le traitement et l'amélioration des symptômes de conditions associé avec une activité excessive de la leucyl aminopeptidase et de conditions associés avec une prolifération excessive de cellules, ces conditions étant le cancer, en particulier, le cancer de la prostate, le cancer colorectal ou la leucémie.

12. Utilisation suivant la revendication 11 dans laquelle le composé suivant la revendication 1 est utilisé dans la fabrication d'un médicament sous forme liquide comme une solution, un sirop ou élixir; une solution ou émulsion stérile; ou sous forme solide comme comprimé, pilule, capsule, granule, comprimé enrobé et poudre.
